# EUROPÄISCHE PATENTSCHRIFT

(11) **EP 1 166 717 B1**
(45) Veröffentlichungstag und Bekanntmachung des Hinweises auf die Patenterteilung: **08.06.2005**
(21) Anmeldenummer: 00113235.6
(22) Anmeldetag: 21.06.2000
(51) Int. Cl.: A61B 5/103, G01N 29/18

(54) **Messgerät zur Messung der elastischen Eigenschaften einer Oberflächenstruktur**
Device for measuring elastic properties of a surface structure
Dispositif pour mesurer les propriétés élastiques d'une structure de surface

(43) Veröffentlichungstag der Anmeldung: 02.01.2002
(73) Patentinhaber: COURAGE + KHAZAKA ELECTRONIC GmbH, D-50829 Köln (DE)
(72) Erfinder: Courage, Wilfried, 50937 Köln (DE)
(74) Vertreter: Dallmeyer, Georg, Dipl.-Ing.

(56) Entgegenhaltungen:
- EP-A- 0 329 817
- WO-A-97/11641
- WO-A-97/25921
- US-A- 5 408 882

## Beschreibung

Die Erfindung betrifft ein Messgerät zur Messung der elastischen Eigenschaften einer Oberflächenstruktur nach dem Oberbegriff des Anspruchs 1. Ein derartiges Messgerät ist aus der EP 0 329 817 bekannt. Das bekannte Gerät weist eine in einem Gehäuse axial beweglich gelagerte Sonde auf, deren Messspitzen mit einem vorgegebenen Anpressdruck orthogonal auf die Hautoberfläche eines biologischen Körpers aufsetzbar sind. Die Messspitzen übertragen Schallimpulse von bimorphen Piezowandlern in die Hautoberfläche, wobei eine Messspitze als Sender arbeitet und die beiden anderen als Empfänger. Die Empfänger messen die Zeitdauer zwischen dem Senden und Empfangen des akustischen Impulses, der zwischen den Messspitzen durch die Haut verläuft. Aus der Zeitmessung wird die Ausbreitungsgeschwindigkeit der Schallwelle in der Haut ermittelt.

Die flachen und streifenförmigen bimorphen Piezowandler bestehen aus einem mit Elektroden versehenen keramischen Material, das eine geringe Bruchfestigkeit aufweist. Die bimorphen Elemente sind daher sehr spröde und brechen schnell bei mechanischer Belastung.

Der Erfindung liegt die Aufgabe zugrunde, ein Messgerät der eingangs genannten Art zu schaffen, das eine höhere Betriebssicherheit aufweist und unempfindlich gegen Stöße ist.

Zur Lösung dieser Aufgabe dienen die Merkmale des Anspruchs 1.

Die Erfindung sieht in vorteilhafter Weise vor, dass die Messspitzen von der Halterung für die bimorphen Elemente gebildet sind, wobei die streifenförmigen bimorphen Elemente nur einseitig mit einem Ende an der Halterung befestigt sind. Dadurch, dass die Halterungen für die bimorphen Elemente selbst die Messspitze bilden, werden die bimorphen Elemente in vorteilhafter Weise keinen mechanischen Belastungen ausgesetzt, so dass sie auch bei unsachgemäßer Behandlung oder bei hoher Stoßbelastung nicht brechen.

Vorzugsweise sind die bimorphen Elemente an dem den Messspitzen zugewandten Ende der Halterung befestigt. Die Halterung ermöglicht die Übertragung von Schallimpulsen über die Messspitzen auf die Oberflächenstruktur und schützt zugleich das bimorphe Element vor Beschädigung. Die streifenförmigen bimorphen Elemente sind jeweils auf einer Halterung an ihrem freien, der Oberflächenstruktur zugewandten Ende, vorzugsweise durch Verkleben befestigt, wodurch sie die erzeugten Schallimpulse optimal auf das freie, die Messspitze bildende Ende der Halterung übertragen können.

An dem freien Ende eines jeden streifenförmigen bimorphen Elementes ist jeweils eine seismische Masse, vorzugsweise durch Verkleben befestigt. Biegt sich das bimorphe Element des Senders infolge eines elektrischen Impulses wird das Biegemoment als Schallimpuls über die Messspitze auf die Oberflächenstruktur übertragen. Als Abstützung für dieses Biegemoment dient die seismische Masse mit ihrer Massenträgheit. Die Massenträgheit der seismischen Masse bewirkt, dass die Anordnung sehr empfindlich auf schnelle Impulse, aber kaum auf relativ langsame Bewegungen, die durch unsachgemäße Behandlung entstehen, reagiert.

Vorzugsweise ist vorgesehen, dass die Halterungen eine den bimorphen Elementen angepasste längliche Aussparung aufweisen, in der die bimorphen Elemente frei schwingen können. Die Aussparung ermöglicht, dass die parallel zu den Halterungen verlaufenden bimorphen Elemente bei ihrer orthogonal zu den Halterungen verlaufenden Schwingung durch die Aussparung hindurchschwingen können.

Die Halterungen bestehen aus zwei flachen, streifenförmigen Teilen, die an den den Messspitzen gegenüberliegenden Enden mit parallelem Abstand zueinander an einem gemeinsamen Träger befestigt sind. Die Halterungen sind sehr flexibel, so dass die Schallimpulse des Senders nicht auf den Träger übertragen werden. Makrolon wird als bevorzugtes Material für die Halterungen verwendet. Die Biegsamkeit der Halterung verhindert dabei, dass der Schallimpuls über die Aufhängung auf den Empfänger übertragen wird.

Die Sonde ist axial beweglich in dem Gehäuse gelagert und die Messspitzen können unter einer definierten Federspannung gegen die Hautoberfläche angelegt werden.

Der Schallimpuls und die Messung können ausgelöst werden, wenn die Sonde einen vorbestimmten Weg zurückgelegt hat. Dadurch ist gewährleistet, dass die Messspitzen stets mit dem gleichen Anpressdruck gegen die Oberflächenstruktur angelegt sind.

Der Schallimpuls und die Messung können auch nach einer vorbestimmten Zeitverzögerung auslösbar sein, wobei dies auch in Kombination mit dem Zurücklegen eines bestimmten Sondenweges möglich ist.

Es ist vorzugsweise vorgesehen, dass der aus einem flachen bimorphen Element bestehende Sender einen Einzelschallimpuls in die Oberflächenstruktur einkoppelt.

Die Auswerteeinrichtung kann die von dem Empfänger aufgenommenen Messsignale mit Hilfe einer Resonanzmessung im Frequenzbereich zwischen 0 und 10 kHz auswerten.

Bei einer Weiterbildung der Erfindung ist vorgesehen, dass die Sonde ein auf der zu untersuchenden Oberflächenstruktur fixierbares Führungsteil aufweist, in dem die Sonde orthogonal zur Oberflächenstruktur geführt ist und in dem die Sonde mit Hilfe von Markierungen an der Sonde und an dem Führungsteil unterschiedliche definierte Winkelstellungen einnehmen kann. Das Führungsteil; das beispielsweise aus einer zylindrischen Hülse besteht, die auf die zu untersuchende Oberflächenstruktur aufgeklebt wird, nimmt die Sonde so auf, dass eine orthogonale Ausrichtung der Sonde relativ zur Oberflächenstruktur gewährleistet ist. Desweiteren sind auf dem Umfang des Führungsteils Winkelmarkierungen angebracht, die es in Verbindung mit einer Markierung an der Sonde ermöglichen, die Sonde um vorbestimmte Winkel zu drehen, so dass Messungen relativ zur einer Ausgangsposition unter einem Winkel von beispielsweise 0°, 45°, 90° oder 135° möglich sind.

Im Folgenden wird unter Bezugnahme auf die Zeichnung ein Ausführungsbeispiel der Erfindung näher erläutert.

Es zeigen:
- Fig. 1: ein Querschnitt durch das Messgerät,
- Fig. 2: eine Draufsicht auf eine Halterung mit einem bimorphen Element, und
- Fig. 3: eine Draufsicht auf ein Führungsteil für die Sonde.

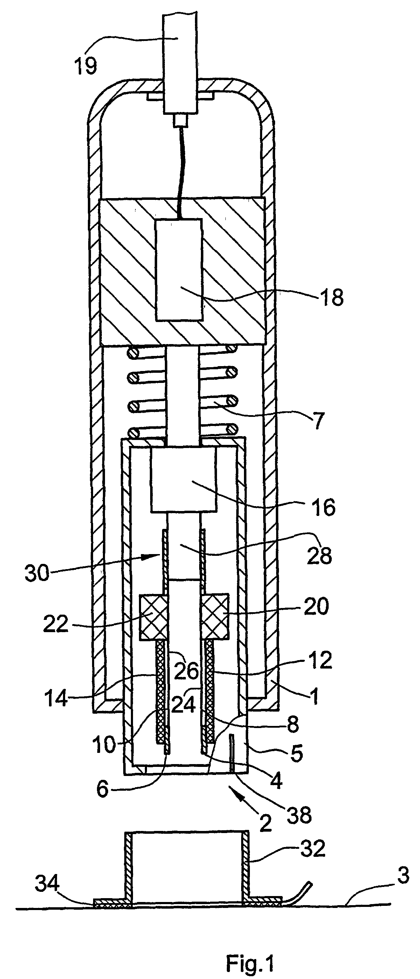

Das in Fig. 1 gezeigte Messgerät besteht aus einem Gehäuse 1, in dem axial beweglich eine Sonde 2 angeordnet ist. Die Sonde 2 besteht aus einem Gehäuse 5, in dem an einem Träger 28 mit parallelem Abstand voneinander zwei flache, streifenförmige sich längs erstreckende Halterungen 8, 10 befestigt sind, die jeweils ein bimorphes Element 12, 14 aufnehmen. Die Halterungen 8, 10 bestehen aus Makrolon und weisen eine hohe Flexibilität auf. Die Halterungen 8, 10 sind nur einseitig an dem Träger 28 befestigt und können mit ihren freien Enden frei schwingen.

Die freien Enden der Halterungen 8, 10 bilden zugleich die Messspitzen 4, 6, auf die Schwingungen eines von dem bimorphen Element 12 erzeugten Schallimpulses auf die zu untersuchende Oberflächenstruktur 3 übertragen werden. Das bimorphe Element 12 erstreckt sich streifenförmig parallel zu der Halterung 8 und ist im Bereich der Messspitze 4 vorzugsweise auf der Außenseite verklebt. Das freie, von der Messspitze 4 abgewandte Ende des bimorphen Elementes 12 trägt eine seismische Masse 20, die vorzugsweise aufgeklebt ist.

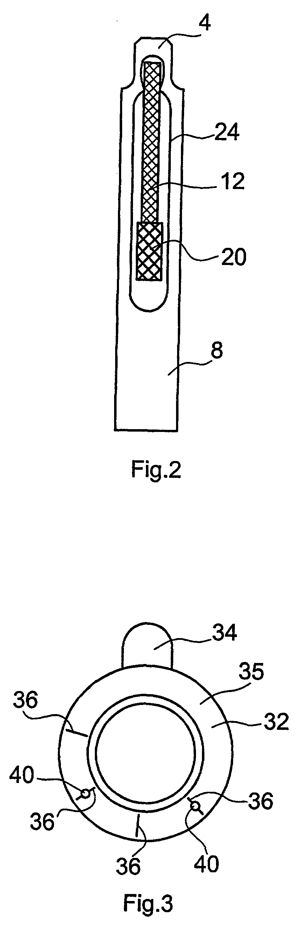

Ein von einer Steuereinrichtung 16 mit einer Leistungselektronik dem bimorphen Element 12 zugeführter elektrischer Impuls erzeugt ein Biegemoment in dem bimorphen Element und überträgt einen Schallimpuls auf die Messspitze 4. Dabei dient die seismische Masse 20 als Momentabstützung. Die Massenträgheit der Masse 20 bewirkt, dass die Anordnung sehr empfindlich auf schnelle Impulse, dagegen kaum empfindlich auf relativ langsame Bewegungen, die durch unsachgemäßen Umgang mit dem Messgerät entstehen, reagiert. Das bimorphe Element 12 bildet mit der Messspitze 4 und der seismischen Masse 20 einen Sender für Schallimpulse. Spiegelbildlich zu der Längsachse des Messgerätes durch den Träger 28 befindet sich ein Empfänger, der aus der Messspitze 6, der Halterung 10, dem bimorphen Element 14 und der seismischen Masse 22 gebildet ist. Die Halterungen 8, 10 weisen, wie am besten aus Fig. 2 ersichtlich ist, eine Aussparung 24, 26 auf, die ein freies Schwingen der bimorphen Elemente 12, 14 und der seismischen Massen 20, 22 in eine orthogonal zu den Halterungen 8, 10 verlaufenden Richtung ermöglicht.

Das Gehäuse 5 und die Messsonde 2 werden innerhalb des Gehäuses 1 mit Hilfe einer Druckfeder 7 vorgespannt.

Eine Auswerteeinrichtung 18 analysiert die von der Messspitze 6 aufgenommenen Messsignale, beispielsweise mit Hilfe einer Resonanzmessung im Frequenzbereich zwischen 0 und 10 kHz.

Die Ergebnisse der Messungen können auf einem Display einer in den Zeichnungen nicht dargestellten Anzeigeeinheit dargestellt werden, die mit der Auswerteeinrichtung 18 über ein Kabel 19 verbunden ist.

Ein hohlzylindrisches Führungsteil 32 kann das distale Ende der Sonde 2 aufnehmen, so dass die Messspitzen 4, 6 exakt orthogonal zur Hautoberfläche 3 während der Messung ausgerichtet sind. Das Führungsteil 32 weist einen Flansch auf, der auf seiner der Oberflächenstruktur 3 zugewandten Seite eine auswechselbare, im wesentlichen ringförmige doppelklebende Folie 34 trägt. Mit Hilfe der doppelklebenden Folie 34 kann das Führungsteil 32 an einer zu untersuchenden Stelle der Oberflächenstruktur 3 fixiert werden.

Wie am besten aus Fig. 3 ersichtlich, weist der Ringflansch 35 auf seinem Umfang zwei Löcher 40 auf, die dazu dienen, auf der Oberflächenstruktur 3, beispielsweise mit einem Stift Farbmarkierungen aufzutragen, um die Messung an gleicher Stelle mit gleicher Orientierung der Sonde 2 auch in größeren Zeitabständen durchführen zu können.

Für die Aussagekraft der Messergebnisse ist es nämlich wesentlich, dass die zu vergleichenden Messergebnisse an der gleichen Stelle der Oberflächenstruktur 3 erfolgen.

Desweiteren trägt der Ringflansch Markierungen 36, die einen vorbestimmten Winkelabstand voneinander haben. In Verbindung mit einer außen an dem Gehäuse 5 angebrachten Markierung 38 der Sonde 2 (Figur 1) lässt sich das Messgerät reproduzierbar an der gleichen Messstelle und in der gleichen Winkelstellung auf der Oberflächenstruktur 3 anordnen.

Auf der Anzeigeeinrichtung können dann Messwerte, beispielsweise unter einem Winkel von 0°, 45°, 90° und 135° angezeigt werden, so dass auch eine Aussage über die Anisotropie der Oberflächenstruktur 3 gemacht werden kann.

Desweiteren ist es möglich, auf der Anzeigeeinheit Durchschnittswerte wiederholter Messungen anzuzeigen. Die Auswerteeinrichtung 18 ist auch in der Lage, Messwerte für einen späteren Vergleich zu speichern.

Die Messung kann ausgelöst werden, wenn die Messspitzen 4, 6 unter einer bestimmten Vorspannung gegen die Hautoberfläche 3 gedrückt werden. Die Höhe der Vorspannung kann beispielsweise über eine Wegmessung der Sonde 2 festgestellt werden, die die Axialverschiebung der Sonde 2 gegen die Federkraft mißt. Zusätzlich kann die Auslösung des Schallimpulses und die Messung zeitlich verzögert werden.

Die verwendeten Schallimpulse liegen im Bereich zwischen 0,5 und 30 kHz.

Die Messwerte sind repräsentativ für die elastischen Eigenschaften der Oberflächenstruktur 3 , bei der auch unterschiedliche Elastizitätswerte für die Quer- und Längselastizität feststellbar sind.

Die Oberflächenstruktur 3 kann aus elastischen schwingungsfähigen Materialien bestehen, deren viskoelastischen Eigenschaft untersucht werden können. Das Messgerät ist auch einsetzbar zur Materialprüfung bei unterschiedlichen Werkstoffen oder zur Produktionkontrolle, beispielsweise bei gummiartigen oder lederartigen Materialien.

Ein wichtiges Anwendungsgebiet derartiger Messung im dermatologischem Bereich sind die Messung der Viskoelastizität und Anisotropie der Haut z.B. für Untersuchung der Auswirkungen von Kosmetika und Pharmazeutika auf Kollagen- und Elastinfasern der Haut, für Untersuchungen der weiblichen Brust und für Untersuchungen im Zusammenhang mit Narbenbehandlungen.

## Patentansprüche

1. Messgerät zur Messung der elastischen Eigenschaften einer Oberflächenstruktur (3) mit einer in einem Gehäuse (1) angeordneten Sonde (2), mit einem Sender und mindestens einem Empfänger, wobei von dem Sender Schallimpulse emittiert und von dem Empfänger das Ausbreitungsverhalten der Schallimpulse in der Oberflächenstruktur (3) erfasst werden, mit einer Steuereinrichtung (16) für die Schallimpulserzeugung und mit einer Auswerteeinrichtung (18) für die von dem Empfänger aufgenommenen Messsignale, wobei mindestens zwei nebeneinander angeordnete auf die Oberflächenstruktur (3) aufsetzbare Messspitzen (4,6) mit von einer Halterung (8,10) getragenen streifenförmigen bimorphen Elementen (12,14) als Sende- und Empfangselemente verbunden sind,
**dadurch gekennzeichnet,**
**dass** die Messspitzen (4,6) von den Halterungen (8,10) für die bimorphen Elemente (12,14) gebildet sind und dass die streifenförmigen bimorphen Elemente (12,14) einseitig mit ihrem einen Ende an der Halterung (8,10) befestigt sind.

2. Messgerät nach Anspruch 1, **dadurch gekennzeichnet, dass** die bimorphen Elemente (12,14) an dem den Messspitzen (4,6) zugewandten Ende der Halterung (8,10) befestigt sind.

3. Messgerät nach Anspruch 1 oder 2, **dadurch gekennzeichnet, dass** an dem freien Ende eines jeden streifenförmigen bimorphen Elementes (12,14) eine seismische Masse (20,22) befestigt ist.

4. Messgerät nach einem der Ansprüche 1 bis 3, **dadurch gekennzeichnet, dass** die Halterungen (8,10) eine den bimorphen Elementen (12,14) angepasste längliche Aussparung (24,26) aufweisen, in der die bimorphen Elemente (12,14) frei schwingen können.

5. Messgerät nach einem der Ansprüche 1 bis 4, **dadurch gekennzeichnet, dass** die Halterungen (8,10) aus zwei flachen streifenförmigen Teilen bestehen, die an dem den Messspitzen (4,6) gegenüberliegenden Ende (30) mit parallelem Abstand zueinander an einem gemeinsamen Träger (28) befestigt sind.

6. Messgerät nach einem der Ansprüche 1 bis 5, **dadurch gekennzeichnet, dass** die Sonde (2) axial beweglich in dem Gehäuse (1) gelagert ist und die Messspitzen (4,6) unter Federspannung gegen die Oberflächen (3) anlegbar sind.

7. Messgerät nach Anspruch 6, **dadurch gekennzeichnet, dass** der Schallimpuls und die Messung nach einem vorbestimmten Weg der Sonde (2) auslösbar sind.

8. Messgerät nach Anspruch 6 oder 7, **dadurch gekennzeichnet, dass** der Schallimpuls und die Messung nach einer vorbestimmten Zeitverzögerung auslösbar sind.

9. Messgerät nach einem der Ansprüche 1 bis 8, **dadurch gekennzeichnet, dass** der aus einem flachen bimorphen Element (12) bestehende Sender einen Einzelschallimpuls in die Oberflächenstruktur einkoppelt.

10. Messgerät nach einem der Ansprüche 1 bis 9, **dadurch gekennzeichnet, dass** die Auswerteeinrichtung (18) die von dem Empfänger aufgenommenen Messsignale mit Hilfe einer Resonanzmessung im Frequenzbereich zwischen 0 und 10 kHz auswertet.

11. Messgerät nach einem der Ansprüche 1 bis 10, **dadurch gekennzeichnet, dass** die Sonde (2) ein auf der Oberflächenstruktur (3) fixierbares Führungsteil(32) aufweist, in dem die Sonde (2) orthogonal zur Oberflächenstruktur (3) gehalten ist und in dem die Sonde (2) mit Hilfe von Markierungen (36,38) an der Sonde (2) und an dem Führungsteil (32) unterschiedliche Winkelstellungen einnehmen kann.

## Claims

1. Measuring device for measuring the elastic properties of a surface structure (3) comprising a probe (2) arranged in a housing (1), a transmitter and at least one receiver, wherein the transmitter emits sound pulses and the receiver detects the propagation behaviour of the sound pulses in the surface structure (3), a control means (16) for sound pulse generation, and an evaluation means (18) for the measuring signals picked up by the receiver, wherein at least two measuring tips (4,6) arranged side by side and adapted to be placed onto the surface structure (3) are connected with strip-shaped bimorph elements (12, 14) configured as transmitter and receiver elements and supported by a holding fixture (8,10),
**characterized in that**
the measuring tips (4,6) are formed by the holding fixtures (8,10) for the bimorph elements (12,14), and the strip-shaped bimorph elements (12,14) are fixed, with one of their ends, on one side to the holding fixture (8,10).

2. Measuring device according to claim 1, **characterized in that** the bimorph elements (12,14) are fixed at the end of the holding fixture (8, 10) facing the measuring tips (4,6).

3. Measuring device according to claim 1 or 2, **characterized in that** at the free end of each strip-shaped bimorph element (12,14) a seismic mass (20,22) is fixed.

4. Measuring device according to one of claims 1 to 3, **characterized in that** the holding fixtures (8,10) comprise an elongate recess (24,26) made to fit the bimorph elements (12,14), in which recess (24,26) the bimorph elements (12,14) are allowed to oscillate freely.

5. Measuring device according to one of claims 1 to 4, **characterized in that** the holding fixtures (8,10) comprise two flat strip-shaped portions fixed, at the end (30) opposite to the measuring tips (4,6) and in parallel relationship to each other, to a common carrier (28).

6. Measuring device according to one of claims 1 to 5, **characterized in that** the probe (2) is supported, in an axially movable manner, in the housing (1), and the measuring tips (4,6) are adapted to be applied under spring tension to the surface (3).

7. Measuring device according to claim 6, **characterized in that** the sound pulse and the measurement are adapted to be triggered after the probe (2) has travelled a predetermined path.

8. Measuring device according to claim 6 or 7, **characterized in that** the sound pulse and the measurement are adapted to be triggered after a predetermined time lag.

9. Measuring device according to one of claims 1 to 8, **characterized in that** the transmitter comprising a flat bimorph element (12) couples an individual sound pulse into the surface structure.

10. Measuring device according to one of claims 1 to 9, **characterized in that** the evaluation means (18) evaluates the measuring signals picked up by the receiver by means of a resonance measurement in the frequency range between 0 and 10 kHz.

11. Measuring device according to one of claims 1 to 10, **characterized in that** the probe (2) comprises a guide part (32) adapted to be fixed at the surface structure (3), in which guide part (32) the probe (2) is held orthogonally to the surface structure (3) and the probe (2) can assume different angular positions with the aid of markings (36,38) provided at the probe (2) and the guide part (32).

## Revendications

1. Dispositif pour mesurer les propriétés élastiques d'une structure de surface (3) avec une sonde (2) placée dans un boîtier (1), lequel dispositif comporte :
un émetteur et au moins un récepteur, des impulsions acoustiques étant émises par l'émetteur et le comportement en propagation des impulsions acoustiques dans la structure de surface (3) étant déterminé par le récepteur,
un dispositif de commande (16) pour la génération d'impulsions acoustiques et
un dispositif (18) d'exploitation des signaux de mesure reçus par le récepteur,
au moins deux pointes de mesure (4, 6), qui peuvent être placées l'une à côté de l'autre sur la structure de surface (3), étant reliées à des éléments bimorphes (12, 14) en forme de bande qui sont supportés par un support (8, 10) et conformés en éléments d'émission et de réception,
**caractérisé en ce que** les pointes de mesure (4, 6) sont formées par les supports (8, 10) destinés aux éléments bimorphes (12, 14) et **en ce que** les éléments bimorphes en forme de bande (12, 14) sont fixés d'un côté, par une extrémité, au support (8, 10).

2. Dispositif de mesure selon la revendication 1, **caractérisé en ce que** les éléments bimorphes (12, 14) sont fixés à l'extrémité du support (8, 10) qui est dirigée vers les pointes de mesure (4, 6).

3. Dispositif de mesure selon la revendication 1 ou 2, **caractérisé en ce qu'**une masse sismique (20, 22) est fixée à l'extrémité libre de chaque élément bimorphe (12, 14) en forme de bande.

4. Dispositif de mesure selon l'une des revendications 1 à 3, **caractérisé en ce que** les supports (8, 10) comporte un évidement oblong (24, 26) qui est adapté aux éléments bimorphes (12, 14) et dans lequel les éléments bimorphes (12, 14) peuvent osciller librement.

5. Dispositif de mesure selon l'une des revendications 1 à 4, **caractérisé en ce que** les supports (8, 10) sont constitués de deux parties plates en forme de bande qui sont fixées, par l'extrémité (30) opposée aux pointes de mesure (4, 6), à un support commun (28) parallèlement à distance l'une de l'autre.

6. Dispositif de mesure selon l'une des revendications 1 à 5, **caractérisé en ce que** la sonde (2) est montée dans le boîtier (1) en étant mobile axialement et les pointes de mesure (4, 6) peuvent être appliquées contre les surfaces (3) en étant contraintes élastiquement.

7. Dispositif de mesure selon la revendication 6, **caractérisé en ce que** l'impulsion acoustique et la mesure peuvent être déclenchées après un trajet prédéterminé de la sonde (2).

8. Dispositif de mesure selon la revendication 6 ou 7, **caractérisé en ce que** l'impulsion acoustique et la mesure peuvent être déclenchées après un retard prédéterminé.

9. Dispositif de mesure selon l'une des revendications 1 à 8, **caractérisé en ce que** l'émetteur, constitué d'un élément bimorphe plat (12), injecte une impulsion acoustique unique dans la structure de surface.

10. Dispositif de mesure selon l'une des revendications 1 à 9, **caractérisé en ce que** le dispositif d'exploitation (18) exploite les signaux de mesure, reçus par le récepteur, à l'aide d'une mesure par résonance dans la plage de fréquences entre 0 et 10 kHz.

11. Dispositif de mesure selon l'une des revendications 1 à 10, **caractérisé en que** la sonde (2) comporte une pièce de guidage (32), qui peut être fixée à la structure de surface (3), dans laquelle la sonde (2) est maintenue perpendiculaire à la structure de surface (3) et dans laquelle la sonde (2) peut prendre différentes positions angulaires à l'aide de repères (36, 38) sur la sonde (2) et sur la pièce de guidage (32).
